**Europäisches Patentamt**

**European Patent Office**

**Office européen des brevets**

(19)

(11) Veröffentlichungsnummer: **0 065 060**
**B1**

# (12) EUROPÄISCHE PATENTSCHRIFT

(45) Veröffentlichungstag der Patentschrift:
**07.01.87**

(21) Anmeldenummer: **81890075.5**

(22) Anmeldetag: **07.05.81**

(51) Int. Cl.⁴: **C 07 C 37/20,** C 07 C 37/70,
C 07 C 39/15

(54) Verfahren zur Herstellung von Phenol-Keton-Kondensationsprodukten, insbesondere von Diphenolen.

(43) Veröffentlichungstag der Anmeldung:
**24.11.82 Patentblatt 82/47**

(45) Bekanntmachung des Hinweises auf die Patenterteilung:
**07.01.87 Patentblatt 87/2**

(84) Benannte Vertragsstaaten:
**AT BE CH DE FR GB IT LI NL SE**

(56) Entgegenhaltungen:
**DE - A - 2 508 710**
**DE - A - 2 928 443**
**DE - A - 3 020 403**
**US - A - 4 024 194**
**US - A - 4 049 721**

(73) Patentinhaber: **ISOVOLTA Österreichische
Isolierstoffwerke Aktiengesellschaft,
Industriezentrum-Süd, A-2351 Wiener Neudorf (AT)**

(72) Erfinder: **Szabolcs, Otto, Dr., c/o Josef Szabolcs
Billrothstrasse 50, A-1190 Wien (AT)**

(74) Vertreter: **Stampfer, Heinz, ISOVOLTA Österreichische
Isolierstoffwerke AG Industriezentrum-Süd,
A-2351 Wiener Neudorf (AT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Diphenolen durch Umsetzung von Ketonen mit Phenolen in einer Kondensationsreaktion, bei welchem in das Reaktionsgemisch Chlorwasserstoffgas als Kondensationsmittel eingeleitet wird, wobei nach Beendigung bzw. Abbruch der Kondensationsreaktion das Gemisch mit Wasser versetzt wird und das erhaltene Kondensationsprodukt dann in einem Reinigungsprozess aufgearbeitet wird. Das erfindungsgemässe Verfahren soll insbesondere bei der Umsetzung von Ketonen anwendbar sein, die in ihren Kohlenwasserstoffresten einen relativ komplexen Aufbau aufweisen.

Ein Verfahren der vorgenannten Art ist aus Beispiel II der US-A-3 546 165 bekannt, in welchem die Herstellung von 9,9-Bis(4-hydroxyphenyl)-fluoren beschrieben ist. Bei diesem Verfahren wird Fluorenon in Phenol gelöst, wonach β-Mercaptoproprionsäure als Katalysator hinzugefügt wird. Danach wird während 10 min Chlorwasserstoffgas in diese Lösung eingeleitet und anschliessend 2 Tage nachreagieren gelassen. Das Rohprodukt, das nach Verdünnung der Lösung mit Wasser als weisser Niederschlag ausfällt, wird dann einem Reinigungsprozess unterzogen.

Eine Analyse mittels Hochdruck-Flüssigkeitschromatographie zeigt, dass das so erhaltene Rohprodukt, ausser dem gewünschten Endprodukt 9,9-Bis(4-hydroxypheny)-fluoren, neben Resten der Ausgangsprodukte noch Nebenprodukte enthält, die als ein Orthoisomeres des Endproduktes und als höher kondensierte Nebenprodukte identifiziert werden können. Bei der Reinigung des Rohproduktes ist nun aber gerade dieses Orthoisomere nur schwer abzutrennen, was dazu führt, dass trotz relativ hoher Rohausbeuten die Reinausbeuten der nach diesem bekannten Verfahren hergestellten Diphenole mit Reinheitsgraden, bei welchen sie vorteilhaft als Monomere bei Polykondensationsreaktionen eingesetzt werden können, relativ gering sind und der dazu benötigte Reinigungsaufwand relativ hoch ist.

Hier will die Erfindung Abhilfe schaffen: Das erfindungsgemässe Verfahren löst die Aufgabe, ein Verfahren zur Herstellung von Diphenolen durch Umsetzung von Ketonen mit Phenolen anzugeben, bei welchem weniger Nebenprodukte entstehen als bei dem vorgenannten bekannten Verfahren und mit Hilfe von dem Diphenole als Reinprodukte bei etwa gleich hoher Reinheit mit einem geringeren Verfahrensaufwand herstellbar sind als es unter Benutzung dieses bekannten Verfahrens möglich ist.

Das wird gemäss dem erfindungsgemässen Verfahren zunächst gemäss Anspruch 1 dadurch erreicht, dass in das zumindest bei Reaktionsbeginn in flüssigem Zustand vorliegende Reaktionsgemisch zwei-, drei- oder vierwertige Metallchloride in relativ zum Keton submolaren Mengen als zusätzliche Kondensationsmittel eingesetzt werden. Die Ansprüche 1 bis 12 betreffen vorteilhafte Ausgestaltungen bzw. Spezialfälle des erfindungsgemässen Verfahrens.

Aus der DE-A-2 508 710 ist nun zwar ein Verfahren zur Herstellung von Trisphenolen bekannt, bei welchem eine Umsetzung von substituierten Acetophenonen mit Phenolen in Gegenwart von sauren Katalysatoren vorgenommen wird, wobei beliebige Katalysatoren wie Mineralsäuren aber auch Lewis-Säuren in Frage kommen sollen. Spezielle vorteilhafte Katalysatorkombinationen wie beim Erfindungsgegenstand sind hier aber nicht vorgesehen.

Das erfindungsgemässe Verfahren hat seine Bedeutung in Phenol-Keton-Kondensationsreaktionen mit Phenolen im weiteren Sinne, seine grössten praktischen Konsequenzen bestehen aber bei Phenol ($C_6H_5OH$) im engeren Sinn.

Nachstehend ist die Produktion von Diphenolen an einer Reihe von Synthesen erläutert, von denen die Synthese I und II im technischen Massstab durchgeführt sind und bei denen zumindest einer der Kohlenwasserstoffreste des eingesetzten Ketons ein Arylrest ist bzw. die Kohlenwasserstoffreste einen zweiwertigen mit der $>C=O$ Gruppe des Ketons verbundenen aromatischen Rest bilden.

Synthese I: Herstellung von 9,9-Bis(4-hydroxyphenyl)-fluoren im technischen Massstab.

In einem mit einem Ankerrührwerk versehenen 250 l Autoklaven werden 50 kg (532 Mol) Phenol geschmolzen und danach 36 kg (200 Mol) Fluorenon und 2,7 kg (20 Mol) $ZnCl_2$ bei 60°C in dem Phenol gelöst. In dieses Reaktionsgemisch wird dann unter ständigem Rühren bei 60°C während einer Zeit von 8 Stunden 2,4 kg (66 Mol) HCl-Gas kontinuierlich eingeleitet und das Reaktionsgemisch danach während 16 Stunden zum Nachreagieren im Autoklaven belassen.

Das Reaktionsgemisch, in dem die Kondensationsreaktion nun völlig abgeschlossen ist und welches in Form eines hellen Kristallbreis vorliegt, wird nun noch im Autoklaven unter Rühren mit heissem Wsser versetzt, wobei die geringen Mengen des sich noch in Lösung befindlichen Reaktionsproduktes ausfallen. Dieses so verdünnte Gemisch wird dann in eine Siebzentrifuge eingebracht, wo das Reaktionsprodukt während des Zentrifugierens mit kochend heissem Wasser gewaschen wird. Dabei werden die Salzsäure, das Zinkchlorid und das überschüssige Phenol ausgewaschen und das zurückbleibende weisse kristalline Produkt im Vakuumtrockenschrank bei etwa 100°C getrocknet.

Die Rohausbeute an dem erhaltenen Diphenol, nämlich dem 9,9-Bis(4-hydroxyphenyl)-fluoren beträgt dabei 67 kg (96% der theoretischen).

Für eine Reinigung dieses Rohproduktes werden in einem heiz- bzw. kühlbaren mit einem Rührwerk versehenen geschlossenen Kessel die erhaltenen 67 kg Roh-Diphenol in ca. 500 l 1,2-Dichloräthan bei Rückflusstemperatur (84°C) gelöst, mit Aktivkohle behandelt, filtriert und die Lösung unter ständigem Rühren auf 0°C abgekühlt, wobei das Diphenol zum grössten Teil wieder auskristallisiert. Das auskristallisierte Diphenol wird nun in einer Siebzentrifuge von der Mutterlauge befreit, während des Zentrifugierens mit kaltem Dichloräthan gewaschen und in einem Vakuumtrockenschrank bei 100°C getrocknet. Diese Art der Reinigung durch einen einmaligen Umkristallisationsvorgang wird dadurch ermöglicht, dass bereits in der Aufarbeitung die nicht umgesetzten Ausgangs- bzw. Hilfsstoffe nahezu vollständig entfernt wurden.

Die Reinausbeute beträgt dabei etwa 60 kg Diphenol (das sind 86% der theoretischen).

Der auf einem Mikroheiztisch nach Kofler bestimmte Schmelzbereich des erhaltenen reinen 9,9-Bis(4-hydroxyphenyl)-fluorens beträgt 228–230°C.

Synthese II: Herstellung von 1,1-Bis(4-hydroxyphenyl)-1-phenyläthan im technischen Massstab.

Das Syntheseverfahren verläuft in gleicher Weise wie bei der Synthese I, nur werden hier statt des Fluorens 24 kg (200 Mol) Acetophenon in dem geschmolzenen Phenol gelöst.

Nach 8 Stunden Haupt- und 16 Stunden Nachreaktionszeit liegt das Reaktionsgemisch in Form eines leicht gefärbten Kristallbreis vor.

Die Aufarbeitung erfolgt analog zur Synthese I und ergibt als Roh-Diphenol 1,1-Bis(4-hydroxyphenyl)-1-phenyläthan in Form eines schwach gefärbten kristallinen Produktes mit einer Ausbeute von 55 kg (95% der theoretischen). Die Reinigung dieser 55 kg Roh-Diphenol erfolgt ebenfalls durch Umkristallisation aus 600 l 1,2-Dichloräthan, so wie im Zusammenhang mit der Synthese I beschrieben.

Die Reinausbeute beträgt dabei 49 kg Diphenol (das sind 84% der theoretischen).

Der so wie bei der Synthese I bestimmte Schmelzbereich des erhaltenen reinen 1,1-Bis(4-hydroxyphenyl)-1-phenyläthans beträgt 189–191°C.

## Patentansprüche

1. Verfahren zur Herstellung von Diphenolen durch Umsetzung von Ketonen mit Phenolen in einer Kondensationsreaktion, bei welchem in das Reaktionsgemisch Chlorwasserstoffgas als Kondensationsmittel eingeleitet wird, wobei nach Beendigung bzw. Abbruch der Kondensationsreaktion das Gemisch mit Wasser versetzt wird und das erhaltene Kondensationsprodukt dann in einem Reinigungsprozess aufgearbeitet wird, dadurch gekennzeichnet, dass in das zumindest bei Reaktionsbeginn in flüssigem Zustand vorliegende Reaktionsgemisch zwei-, drei- oder vierwertige Metallchloride in relativ zum Keton submolaren Mengen als zusätzliche Kondensationsmittel eingesetzt werden.

2. Verfahren nach Anspruch 1, dadurch gekennzeichnet, dass die Reaktion unter Einleitung des Chlorwasserstoffgases bei Temperaturen zwischen 20 und 85°C, vorzugsweise zwischen 20 und 75°C erfolgt.

3. Verfahren nach Anspruch 1 oder 2, dadurch gekennzeichnet, dass die pro Mol Keton eingesetzte Menge an Metallchloriden zwischen 0,03 bis 0,5 Mol, vorzugsweise aber zwischen 0,1 bis 0,3 Mol beträgt.

4. Verfahren nach einem der Ansprüche 1 bis 3, dadurch gekennzeichnet, dass als zweiwertige Metallchloride $CaCl_2$ und/oder $ZnCl_2$ eingesetzt werden.

5. Verfahren nach einem der Ansprüche 1 bis 3, dadurch gekennzeichnet, dass als dreiwertige Metallchloride $AlCl_3$ und/oder $FeCl_3$ eingesetzt werden.

6. Verfahren nach einem der Ansprüche 1 bis 3, dadurch gekennzeichnet, dass als vierwertige Metallchloride $SnCl_4$ und/oder $TiCl_4$ eingesetzt werden.

7. Verfahren nach einem der Ansprüche 1 bis 6, dadurch gekennzeichnet, dass pro Mol Keton mehr als zwei Mol des Phenols eingesetzt werden, wobei das Keton und das Metallchlorid in dem Phenol gelöst sind.

8. Verfahren nach einem der Ansprüche 1 bis 7, dadurch gekennzeichnet, dass beim Reinigungsprozess zur Abtrennung des überschüssigen Phenols Wasserdampf durch das Reaktionsgemisch geleitet wird.

9. Verfahren nach einem der Ansprüche 1 bis 8, dadurch gekennzeichnet, dass das Roh-Kondensationsprodukt zu seiner Reinigung in einem chlorierten Kohlenwasserstoff als Lösungsmittel, gegebenenfalls unter Zusatz von Aktivkohle, gelöst und aus diesem bei niedriger Temperatur auskristallisiert wird und dass das auskristallisierte Produkt hernach von der Mutterlauge getrennt, mit kaltem Lösungsmittel gewaschen und getrocknet wird.

10. Verfahren nach Anspruch 9, dadurch gekennzeichnet, dass als Lösungsmittel 1,2-Dichloräthan eingesetzt wird.

11. Verfahren nach einem der Ansprüche 1 bis 10, dadurch gekennzeichnet, dass als Keton Fluorenon eingesetzt wird.

12. Verfahren nach einem der Ansprüche 1 bis 10, dadurch gekennzeichnet, dass als Keton Acetophenon eingesetzt wird.

## Claims

1. Process for the preparation of diphenols by reacting ketones with phenols in a condensation reaction, wherein hydrogen chloride gas is passed as condensing agent into the reaction mixture, water being added to the mixture after the completion or termination of the condensation reaction and the condensation product obtained then being worked up in a purification process, characterized in that divalent, trivalent or tetravalent metal chlorides are, in quantities which are submolar relative to the ketone, introduced as additional condensing agents into the reaction mixture which is in the liquid state at least at the start of the reaction.

2. Process according to Claim 1, characterized in that the reaction is carried out with passing the hydrogen chloride gas in at temperatures between 20 and 85°C, preferably between 20 and 75°C.

3. Process according to Claim 1 or 2, characterized in that the quantity of metal chlorides introduced per mol of ketone is between 0.03 and 0.5 mol, and preferably between 0.1 and 0.3 mol.

4. Process according to one of Claims 1 to 3, characterized in that $CaCl_2$ and/or $ZnCl_2$ are used as the divalent metal chlorides.

5. Process according to one of Claims 1 to 3, characterized in that $AlCl_3$ and/or $FeCl_3$ are used as the trivalent metal chlorides.

6. Process according to one of Claims 1 to 3, characterized in that $SnCl_4$ and/or $TiCl_4$ are used as the tetravalent metal chlorides.

7. Process according to one of Claims 1 to 6, characterized in that more than two mol of the phenol are used per mol of ketone, the ketone and the metal chloride being dissolved in the phenol.

8. Process according to one of Claims 1 to 7, characterized in that steam is passed through the reaction mixture for removal of the excess phenol during the purification process.

8. Process according to one of Claims 1 to 8, characterized in that, for purification, the crude condensation product is dissolved in a chlorinated hydrocarbon as a solvent, if appropriate with addition of activated carbon, and is crystallized out of this solvent at low temperature, and that the product which has crystallized out is afterwards separated from the mother liquor, washed with cold solvent and dried.

10. Process according to Claim 9, characterized in that 1,2-dichloroethane is used as the solvent.

11. Process according to one of Claims 1 to 10, characterized in that fluorenone is used as the ketone.

12. Process according to one of Claims 1 to 10, characterized in that acetophenone is used as the ketone.

**Revendications**

1. Procédé de préparation de diphénols par réaction de cétones et de phénols dans une réaction de condensation consistant à ajouter au mélange réactionnel de l'acide chlorhydrique gazeux, agissant comme agent de condensation, et, après achèvement ou interruption de la réaction de condensation, à ajouter de l'eau au mélange résultant puis à soumettre le produit de condensation obtenu à une étape d'épuration, caractérisé en ce qu'on introduit dans le mélange réactionnel se trouvant à l'état liquide au moins au début de la réaction, des chlorures métalliques bivalents, trivalents ou tétravalents, en quantités submolaires par rapport aux cétones, et agissant comme agents additionnels de condensation.

2. Procédé selon la revendication 1, caractérisé en ce que la réaction est mise en œuvre par addition de l'acide chlorhydrique gazeux, à des températures comprises entre 20 et 85 °C, de préférence entre 20 et 75 °C.

3. Procédé selon la revendication 1 ou 2, caractérisé en ce que la quantité de chlorures métalliques introduite par mole de cétone est comprise entre 0,03 et 0,5 mole, et de préférence entre 0,1 et 0,3 mole.

4. Procédé selon l'une des revendications 1 à 3, caractérisé en ce qu'on utilise $CaCl_2$ et/ou $ZnCl_2$ comme chlorure métallique bivalent.

5. Procédé selon l'une des revendications 1 à 3, caractérisé en ce qu'on utilise $AlCl_3$ et/ou $FeCl_3$ comme chlorure métallique trivalent.

6. Procédé selon l'une des revendications 1 à 3, caractérisé en ce qu'on utilise $SnCl_4$ et/ou $TiCl_4$ comme chlorure métallique tétravalent.

7. Procédé selon l'une des revendications 1 à 6, caractérisé en ce qu'on utilise par mole de cétone plus de deux moles de phénol, la cétone et le chlorure métallique étant dissous dans le phénol.

8. Procédé selon l'une des revendications 1 à 7, caractérisé en ce que, dans l'étape d'épuration, en vue de la séparation du phénol en excès, on introduit de la vapeur d'eau dans le mélange réactionnel.

9. Procédé selon l'une des revendications 1 à 8, caractérisé en ce que le produit de condensation brut est dissous, en vue de son épuration, dans un hydrocarbure chloré servant de solvant, le cas échéant avec addition de charbon actif, et est séparé de celui-ci à plus basse température par cristallisation et en ce que le produit ainsi cristallisé est séparé de la lessive-mère et lavé avec un solvant froid puis est séché.

10. Procédé selon la revendication 9, caractérisé en ce qu'on utilise comme solvant du 1,2-dichloréthane.

11. Procédé selon l'une des revendications 1 à 10, caractérisé en ce qu'on utilise comme cétone de la fluorénone.

12. Procédé selon l'une des revendications 1 à 10, caractérisé en ce qu'on utilise comme cétone de l'acétophénone.